# DEMANDE DE BREVET EUROPEEN

(11) **EP 1 994 903 A1**
(43) Date de publication de la demande: **26.11.2008**
(21) Numéro de dépôt: 08102652.8
(22) Date de dépôt: 17.03.2008
(51) Int. Cl.: A61B 17/74, A61B 17/80

(54) **Matériel d'ostéosynthèse du type "vis-plaque**

(30) Priorité: 22.05.2007 FR 0703619
(71) Demandeur: Groupe Lepine, 69394 Lyon Cedex 03 (FR)
(72) Inventeur: Burdin, Philippe, 37320 Branchs (FR); Ropars, Mickael, 35000 Rennes (FR); Galaud, Bertrand, 14000 Caen (FR)
(74) Mandataire: Jeannet, Olivier

(57) **Abrégé**

Ce matériel (11) comprend un canon (12) destiné à être engagé dans le col de l'os et une plaque (13) destinée à être fixée à l'os afin de maintenir le canon (12) en position, le canon (12) et la plaque (13) formant deux pièces séparées et la plaque (13) comprenant un trou (14) de réception du canon (12).

Selon l'invention,
- la plaque (13) se prolonge longitudinalement au-delà de la zone comprenant le trou (14), du côté opposé à la partie diaphysaire de cette plaque (13), et comprend un trou taraudé de réception d'une vis de serrage (17), aménagé dans cette partie prolongée (13b), dont l'axe est sensiblement perpendiculaire à l'axe longitudinal de la plaque (13) et se trouve dans le même plan que l'axe du trou (14) de réception du canon (12) ; et,
- le matériel (11) comprend une vis de serrage (17) destinée à être engagée dans ledit trou taraudé et à venir porter contre la paroi du canon (12) lorsqu'elle est serrée.

## Description

La présente invention concerne un matériel d'ostéosynthèse du type "vis-plaque".

Un tel matériel, bien connu en lui-même, est destiné à l'ostéosynthèse de la tête d'un os, particulièrement de la tête du fémur après fracture du col du fémur. II comprend un canon destiné à être engagé dans le col de l'os, coaxialement à celui-ci, depuis le côté externe de cet os, et une plaque destinée à être fixée à la corticale externe de l'os afin de maintenir le canon en position. Le canon est destiné à recevoir une vis d'ostéosynthèse insérée dans la tête de l'os, comprenant un alésage taraudé proximal dans lequel peut être vissée une vis de recul prenant appui sur la plaque, ce vissage permettant de faire reculer la vis d'ostéosynthèse et donc de réaliser l'ostéosynthèse de la tête de l'os.

Le canon est généralement solidaire de la plaque, formant corps avec celle-ci.

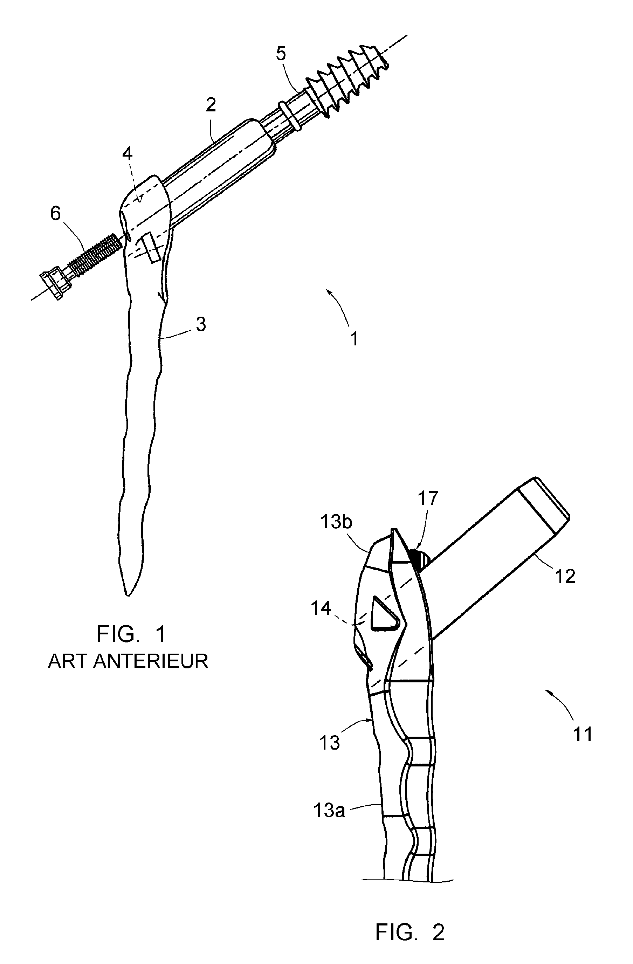

Par la demande de brevet français n° 02 08123, dont la figure 1 est reproduite en figure 1 annexée, il a été divulgué un tel matériel 1 dans lequel le canon 2 forme une pièce séparée de la plaque 3 et peut être relié rigidement à celle-ci, la plaque 3 comprenant un trou 4 de réception et de montage du canon 2 sur elle. La figure 1 montre également la vis d'ostéosynthèse 5 et la vis de recul 6.

Le matériel selon cette demande de brevet apporte une amélioration importante en ce qui concerne la mise en place du matériel 1, du fait de cette structure du canon 2 et de la plaque 3 en deux pièces séparées.

La liaison entre ces deux pièces doit toutefois être parfaitement rigide et parfaitement résistante aux sollicitations répétées que subit un tel matériel après implantation. Le matériel selon ladite demande de brevet n'est pas parfait de ce point de vue.

La présente invention vise à remédier à cet inconvénient essentiel, dans les meilleures conditions.

À cet effet, dans le matériel qu'elle concerne,
- la plaque se prolonge longitudinalement au-delà de la zone comprenant le trou de réception du canon, du côté opposé à la partie diaphysaire de cette plaque, et comprend un trou taraudé de réception d'une vis de serrage, aménagé dans cette partie prolongée, dont l'axe est sensiblement perpendiculaire à l'axe longitudinal de la plaque et se trouve dans le même plan que l'axe du trou de réception du canon ; et,
- le matériel comprend une vis de serrage destinée à être engagée dans ledit trou taraudé et à venir porter contre la paroi du canon lorsqu'elle est serrée.

Cette vis de serrage permet ainsi de parfaitement immobiliser le canon par rapport à la plaque, assurant une liaison rigide et résistante de celui-ci à cette dernière, à même de parfaitement résister aux contraintes répétées que le matériel subit après implantation.

De préférence, l'extrémité distale de la vis de serrage est de forme conique, ayant un angle au sommet complémentaire de l'angle que forme l'axe du canon avec l'axe longitudinal de la plaque.

La paroi de cette extrémité vient ainsi au contact de la paroi du canon selon une surface, permettant de répartir la surface d'appui de la vis de serrage contre le canon. Cette répartition permet non seulement d'obtenir une parfaite liaison de la vis de serrage et du canon mais également d'éviter de former un point de concentration des contraintes et donc d'amorce de rupture.

Dans le même but d'éviter un tel point de concentration, la pointe du cône que forme l'extrémité distale de la vis de serrage, et/ou l'angle formé par la base de ce cône avec le reste du corps de la vis de serrage, est arrondi.

Le filet de la vis de serrage s'interrompt avantageusement à distance de la base du cône que forme l'extrémité distale de la vis de serrage, de sorte que le corps de vis de serrage présente une portion cylindrique dépourvue de filet entre l'extrémité distale du filet et la base du cône que forme l'extrémité distale de la vis de serrage.

Cette interruption permet également d'éviter tout point d'appui ponctuel de la vis de serrage contre le canon et également d'éviter de léser le filet, conservant la possibilité de dévisser la vis de serrage si nécessaire, par exemple à l'occasion d'une reprise.

Selon une possibilité, la paroi du canon comporte une empreinte de forme complémentaire de celle de l'extrémité distale de la vis de serrage.

Cette empreinte permet d'augmenter encore la surface de contact de la vis de serrage avec le canon.

De préférence, ledit trou taraudé de réception de la vis de serrage comprend un lamage proximal permettant de "noyer" la tête de la vis de serrage.

Cette tête ne fait pas saillie par rapport à la plaque et ne constitue donc pas une zone risquant d'être agressive pour les tissus environnants.

La vis de serrage comprend de préférence une cavité de manoeuvre à six pans, permettant d'assurer un serrage intense de cette vis sans risque d'échappement de l'instrument permettant de manoeuvrer cette vis.

L'invention sera bien comprise, et d'autres caractéristiques et avantages de celle-ci apparaîtront, en référence au dessin schématique annexé, représentant, à titre d'exemple non limitatif, une forme de réalisation préférée du matériel d'ostéosynthèse qu'elle concerne.
La figure 2 est une vue partielle, de côté, de la plaque et du canon et d'une vis de serrage qu'il comprend ;
la figure 3 est une vue de côté, à échelle agrandie, de cette vis de serrage ;
la figure 4 est une vue similaire à la figure 2, en coupe longitudinale médiane, et
la figure 5 est une vue similaire à la figure 4, selon une variante de réalisation.

Le figure 2 représente la plaque osseuse 13 et le canon 12 d'un matériel d'ostéosynthèse 11 du type "vis-plaque". Elle représente également une vis de serrage 17 que comprend ce matériel 11.

Le matériel 11 est destiné à l'ostéosynthèse de la tête d'un os, particulièrement de la tête du fémur après fracture du col du fémur. Le canon 12 est destiné à être engagé dans le col de l'os, coaxialement à celui-ci, depuis le côté externe de cet os, et la plaque 13 est destinée à être fixée à la corticale externe de l'os afin de maintenir le canon 12 en position. Le canon 12 est destiné à recevoir une vis d'ostéosynthèse et une vis de recul (non représentées) identiques ou similaires aux vis 4 et 5 précitées.

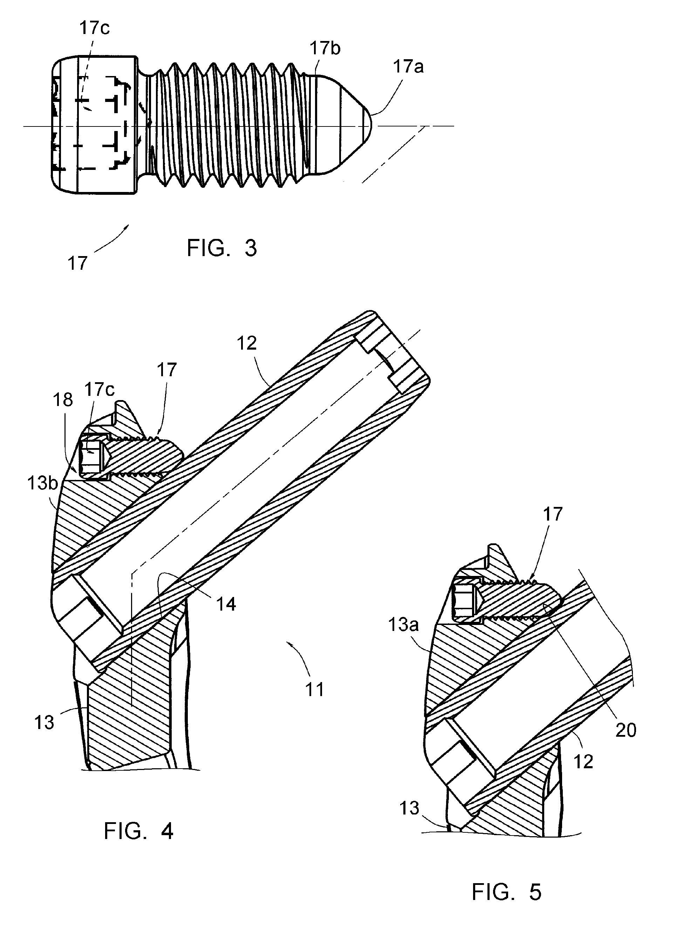

Dans le matériel 11, ainsi que cela apparaît plus particulièrement sur la figure 4, le canon 12 forme une pièce séparée de la plaque 13. Cette dernière comprend à cet effet un trou 14 de réception et de montage du canon 12 sur elle, le canon 12 et la plaque 13 comprenant des moyens pour ce montage. Ces moyens de montage peuvent par exemple être des formes complémentaires de type baïonnette, venant en prise par pivotement angulaire du canon 12 par rapport à la plaque 13, comme décrit par la demande de brevet français n° 02 08123.

II apparaît sur les figures que, par rapport à la plaque 3 existante, la plaque 13 se prolonge longitudinalement au-delà de la zone comprenant le trou 14 de réception du canon 12, du côté opposé à la partie diaphysaire 13a de cette plaque, pour former une partie prolongée 13b. Cette partie prolongée 13b comprend un trou taraudé de réception de la vis 17, dont l'axe est sensiblement perpendiculaire à l'axe longitudinal de la plaque 13 et se trouve dans le même plan que l'axe du trou 14 de réception du canon 12.

Ce trou taraudé comprend un lamage proximal 18 permettant de "noyer" la tête de la vis 17 lorsque celle-ci est serrée, ainsi que le montre la figure 4.

En référence aux figures 3 et 4, il apparaît que la vis 17 comprend une extrémité distale 17a de forme conique, ayant un angle au sommet complémentaire de l'angle que forme l'axe du canon 12 avec l'axe longitudinal de la plaque 13. La pointe du cône que forme cette extrémité distale 17a est arrondi, de même que l'angle formé par la base de ce cône avec le reste du corps de la vis 17.

Il apparaît également que le filet de la vis 17 s'interrompt à distance de la base du cône que forme l'extrémité 17a, de sorte que la vis 17 présente une zone 17b substantiellement cylindrique, dépourvue de filet, adjacente à l'extrémité 17a.

La vis 17 comprend en outre une cavité de manoeuvre 17c à six pans.

En pratique, comme cela se comprend, la vis de serrage 17 est destinée à venir porter contre la paroi du canon 12 lorsqu'elle est serrée, permettant ainsi de parfaitement immobiliser le canon 12 par rapport à la plaque 13. La liaison rigide et résistante ainsi obtenue est à même de parfaitement résister aux contraintes répétées que le matériel 11 subit après implantation.

Le cône que forme l'extrémité 17a permet à cette extrémité 17a de venir au contact de la paroi du canon 12 selon une surface d'appui répartie de la vis 17 contre le canon 12. Cette répartition permet non seulement d'obtenir une parfaite liaison de la vis 17 et du canon 12 mais également d'éviter de former un point de concentration des contraintes et donc d'amorce de rupture. La pointe arrondie de l'extrémité 17a et l'arrondi de l'angle formé par la base du cône de cette extrémité 17a avec la zone 17b contribuent à ce même résultat.

L'interruption du filet de la vis 17 à distance de la base du cône que forme l'extrémité 17a permet également d'éviter tout point d'appui ponctuel de la vis 17 contre le canon 12 et également d'éviter de léser le filet, conservant la possibilité de dévisser la vis de serrage si nécessaire, par exemple à l'occasion d'une reprise.

La cavité 17c permet quant à elle d'assurer un serrage intense de la vis 17 sans risque d'échappement de l'instrument permettant de manoeuvrer cette vis.

La figure 5 montre une variante de réalisation dans laquelle la paroi du canon 12 comporte une empreinte 20 de forme complémentaire de celle de l'extrémité distale 17a de la vis de serrage 17. Cette empreinte 20 permet d'augmenter encore la surface de contact de la vis 17 avec le canon 12.

Comme cela apparaît de ce qui précède, l'invention fournit un matériel d'ostéosynthèse du type "vis-plaque", présentant, par rapport aux matériels homologues de la technique antérieure, l'avantage déterminant d'avoir une structure du canon 12 et de la plaque 13 en deux pièces séparées tout en ayant, entre ces deux pièces, une liaison parfaitement rigide et résistante aux sollicitations répétées que subit un tel matériel après implantation.

II va de soi que l'invention n'est pas limitée à la forme de réalisation décrite ci-dessus à titre d'exemple mais qu'elle s'étend à toutes les formes de réalisation couvertes par les revendications ci-annexées.

## Revendications

1. Matériel d'ostéosynthèse (11) du type "vis-plaque", comprenant un canon (12) destiné à être engagé dans le col de l'os, coaxialement à celui-ci, et une plaque (13) destinée à être fixée à l'os afin de maintenir le canon (12) en position, le canon (12) et la plaque (13) formant deux pièces séparées et la plaque (13) comprenant un trou (14) de réception et de montage du canon (12) sur elle, **caractérisé en ce que** :
- la plaque (13) se prolonge longitudinalement au-delà de la zone comprenant le trou (14) de réception du canon (12), du côté opposé à la partie diaphysaire (13a) de cette plaque (13), et comprend un trou taraudé de réception d'une vis de serrage (17), aménagé dans cette partie prolongée (13b), dont l'axe est sensiblement perpendiculaire à l'axe longitudinal de la plaque (13) et se trouve dans le même plan que l'axe du trou (14) de réception du canon (12) ; et,
- le matériel (11) comprend une vis de serrage (17) destinée à être engagée dans ledit trou taraudé et à venir porter contre la paroi du canon (12) lorsqu'elle est serrée.

2. Matériel (11) selon la revendication 1, **caractérisé en ce que** l'extrémité distale (17a) de la vis de serrage (17) est de forme conique, ayant un angle au sommet complémentaire de l'angle que forme l'axe du canon (12) avec l'axe longitudinal de la plaque (13).

3. Matériel (11) selon la revendication 1 ou la revendication 2, **caractérisé en ce que** la pointe du cône que forme l'extrémité distale (17a) de la vis de serrage (17) est arrondi.

4. Matériel (11) selon l'une des revendications 1 à 3, **caractérisé en ce que** l'angle formé par la base du cône que forme l'extrémité distale (17a) de la vis de serrage (17) avec le reste du corps de la vis de serrage (17), est arrondi.

5. Matériel (11) selon l'une des revendications 1 à 4, **caractérisé en ce que** le filet de la vis de serrage (17) s'interrompt à distance de la base du cône que forme l'extrémité distale (17a) de la vis de serrage (17).

6. Matériel (11) selon l'une des revendications 1 à 5, **caractérisé en ce que** la paroi du canon (12) comporte une empreinte (20) de forme complémentaire de celle de l'extrémité distale (17a) de la vis de serrage (17).

7. Matériel (11) selon l'une des revendications 1 à 6, **caractérisé en ce que** ledit trou taraudé de réception de la vis de serrage (17) comprend un lamage proximal (18) permettant de "noyer" la tête de la vis de serrage (17).

8. Matériel (11) selon l'une des revendications 1 à 7, **caractérisé en ce que** la vis de serrage (17) comprend une cavité de manoeuvre (17c) à six pans.
